# EUROPEAN PATENT APPLICATION

(11) **EP 3 643 235 A1**
(43) Date of publication of application: **29.04.2020**
(21) Application number: 18201838.2
(22) Date of filing: 22.10.2018
(51) Int. Cl.: A61B 5/11, A61B 5/00

(54) **DEVICE, SYSTEM AND METHOD FOR MONITORING A SUBJECT**

(71) Applicant: Koninklijke Philips N.V., 5656 AG Eindhoven (NL)
(72) Inventor: FALCK, Thomas Maria, 5656 AE Eindhoven (NL); VAN DEN HEUVEL, Teun, 5656 AE Eindhoven (NL); GRASSI, Angela, 5656 AE Eindhoven (NL)
(74) Representative: de Haan, Poul Erik

(57) **Abstract**

The present invention relates to a device, system and method for monitoring a subject. To obtain increased performance at reasonable cost and complexity, the device comprises an input interface (81) configured to obtain image data acquired over time by an imaging unit from a field of view containing at least part of a subject, a monitoring signal determination unit (82) configured to determine a monitoring signal from the obtained image data of the subject, a processing unit (83) configured to evaluate the image data to determine the spatial configuration of the subject with respect to the imaging unit and to generate a feedback signal for providing feedback about the determined spatial configuration, and an output interface (84) configured to output the feedback signal.

## Description

### FIELD OF THE INVENTION

The present invention relates to a device, system and method for monitoring a subject.

### BACKGROUND OF THE INVENTION

Camera-based contactless technique provides the possibility to remotely monitor a subject, e.g. a patient in a hospital or an elderly person in a rest home or at home, in an unobtrusive way. Camera-based contactless patient monitoring applications make use of a camera having a suitable view of the patient body or body parts.

The camera view can be hampered by several factors, including one or more of improper position of the patient (and/or his support, such as a bed or chair) in relation to the camera's field of view (FOV), occlusions caused by objects in between camera and patient, and occlusions caused by people (e.g. medical staff or visitors) in between camera and patient.

There are different methods known to address these challenges. One option is to visually check the image data (video output) and take corrective action (which may involve adjusting the camera position and/or angle, and/or moving objects or people), which, however, involves privacy concerns and requires skill and effort. Another option is to use specific workflow instructions on where to place the bed, objects, etc., which is, however, prone to human error and not flexible. Still another option is to provide redundancy through multiple cameras, which, however, increases cost and system complexity.

Hence, there is a need for patient monitoring with increased performance and without one or more of the disadvantages of the known devices and method for patient monitoring by use of image data acquired by a camera.

### SUMMARY OF THE INVENTION

It is an object of the present invention to provide a device, system and method for monitoring a subject with increased performance at reasonable cost and complexity.

In a first aspect of the present invention a device for monitoring a subject is presented comprising:
- an input interface configured to obtain image data acquired over time by an imaging unit from a field of view containing at least part of a subject,
- a monitoring signal determination unit configured to determine a monitoring signal from the obtained image data of the subject,
- a processing unit configured to evaluate the image data to determine the spatial configuration of the subject with respect to the imaging unit and to generate a feedback signal for providing feedback about the determined spatial configuration, and
- an output interface configured to output the feedback signal.

In a further aspect of the present invention a system for monitoring a subject is presented comprising:
- an imaging unit configured to acquire image data over time from a field of view containing at least part of a subject, and
- a device defined in any one of the preceding claims for determining a monitoring signal of a subject based on the acquired image data.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to overcome the disadvantages of known devices, systems and methods by using the image data, which are obtained (i.e. retrieved or received, e.g. directly from an imaging unit or from a storage) and which are used for the monitoring task, to determine (e.g. estimate or compute) the spatial configuration of the subject with respect to the imaging unit. For instance, the relative position and/or orientation of the subject with respect to the field of view of the imaging unit is determined to detect e.g. an improper positioning of the subject and/or the imaging unit. Further, any occlusions of the subject caused e.g. by other objects and/or persons may be detected. Corresponding feedback (e.g. recommendations, instructions, control signals, etc.) may then be generated based on this information, for instance to inform a user about the current spatial relationship and optionally prompt appropriate changes (e.g. automatically and/or through user interaction) to overcome any disadvantages with respect to the actual monitoring tasked caused by current spatial relationship.

Hence, with the present invention it is possible to identify any mispositioning of the imaging unit with respect to the subject and to provide information for achieving a correct positioning of the imaging unit so that the target (i.e. the subject's body that may lie on a bed) is included in the imaging unit's field of view.

The imaging unit may be a conventional camera, e.g. a video camera, still image camera, a Web cam, an RGB camera, etc., that acquires conventional camera images of the field of view. Alternatively, the imaging unit may be a 3D camera, such as a time-of-flight camera to acquire 3D image data including distance information.

The present invention may be used in different monitoring applications and there are different options for the monitoring signal depending on the application. In one application the invention may be used in the context of vital signs monitoring using a conventionally known method, such as respiration measurement from the observations of the movements of the chest and/or belly portion or photoplethysmography (PPG), in which PPG signals are derived from a time-sequence of image data, from which a vital sign (e.g. heart rate, SpO2, respiration rate, etc.) may be derived using a conventionally known methods. In another application activity levels of subjects may be measured and/or unusual motoric behavior of patients (e.g. carphology or floccillation, i.e. lint-picking behavior) may be determined as a symptom of a delirious state of the subject. Generally, the invention may be used for various camera-based applications.

According to an embodiment the processing is configured to evaluate the image data to detect if the subject is visible in the image data or if the subject is occluded or out of the field of view of the imaging unit. For instance, shadows (caused by temporally illuminating the field of view) that cover part of the subject in the image data may be detected to find occlusions, which can then be used to generate corresponding feedback, optionally with instructions how to avoid such occlusions. In another embodiment, e.g. if a 3D (depth) camera is used as imaging unit so that depth information within the image data or along with the image data is available to the device, a projection unit and temporal illumination of the field of view are not required since occlusions and occluding objects can be directly detected from such image data and depth information.

According to another embodiment the field of view is at least temporarily illuminated from substantially the same direction from which the imaging unit views the field of view. Preferably, the field of view is at least temporarily illuminated by a projection unit having a field of projection that substantially overlaps with the field of view of the imaging unit. For instance, a projector may be positioned to have a field of projection substantially overlapping with the field of view of the imaging unit. In an embodiment a projector, e.g. an array of LEDs or other light sources, may be integrated into or mounted at a camera used as imaging unit. In this way, the projector is used to give instructions and feedback about spatial configurations by creating light patterns and/or shadows.

As mentioned above, the feedback may take different forms and comprise different information. In one embodiment the feedback signal may be configured to control a projection unit to indicate through illumination of the field of view if the spatial configuration can be maintained or shall be changed. For instance, illumination of selected areas in the field of view and/or projection of one or more of symbols, colors, letters, and text may be used for this purpose. Different areas of the field of view may thus e.g. be illuminated in different colors and/or different light patterns and/or different luminance to indicate spatial areas of the scene that are well visible in the imaging unit's field of view and other spatial areas that are e.g. occluded.

In another embodiment the feedback signal may be configured to control a user interface to indicate through user feedback if the spatial configuration can be maintained or shall be changed, e.g. by use of visual and/or audio feedback. The user interface may e.g. be a display screen or a loudspeaker arranged next to the scene.

In still another embodiment the feedback signal may be configured to control the projection unit and/or the user interface to indicate how the spatial configuration shall be changed, i.e. in the form of instructions or symbols.

The feedback signal may further be configured to control the projection unit and/or the user interface to indicate i) if and/or how an occluding object shall be moved and/or ii) if and/or how the subject and/or a support of the subject shall be moved and/or iii) if and/or how the imaging unit and/or the projection unit shall be moved. Based on this information the user can take appropriate action to overcome any problems caused by the current spatial configuration.

In another embodiment the processing unit may be configured to generate a control signal for controlling the imaging unit and/or a support for supporting the subject based on the determined spatial configuration to change its position and/or orientation in order to change the spatial configuration, wherein output unit is configured to output the control signal. Thus, an automated system may be realized in which changes of the spatial configuration are automatically effected.

The presented system at least comprises the above described device and an imaging unit for acquiring the image data. Further, the system may comprise a projection unit configured to at least temporarily illuminate the field of view, the projection unit having a field of projection that substantially overlaps with the field of view of the imaging unit, and/or to indicate through illumination of the field of view if the spatial configuration can be maintained or shall be changed, and/or a user interface configured to indicate through user feedback if the spatial configuration can be maintained or shall be changed.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a conventional system,
Fig. 2 shows a schematic diagram of a first embodiment of a system according to the present invention,
Fig. 3 shows a schematic diagram of an embodiment of a device according to the present invention,
Fig. 4 shows a schematic diagram of a second embodiment of a system according to the present invention,
Fig. 5 shows a schematic diagram of a third embodiment of a system according to the present invention, and
Fig. 6 shows a flow chart of an exemplary implementation of a method according to the present invention.

### DETAILED DESCRIPTION OF EMBODIMENTS

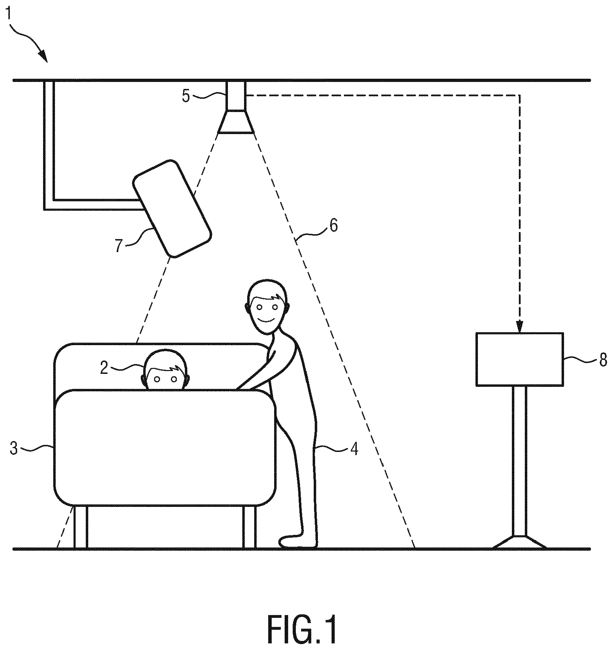

Fig. 1 shows a schematic diagram of a conventional system 1 for monitoring a subject 2. In this scenario the subject 2 is patient, e.g. in a hospital or a rest home, lying in a bed 3. Another person 4, e.g. a nurse or a visitor, is standing by the bed 3. The system 1 comprises a imaging unit 5, such as a conventional optical video camera mounted at the ceiling above the bed 3, which is configured to acquire image data over time from a field of view 6 containing at least part of the patient 2. The acquired image may simply be used for display on a monitor, e.g. in a central monitoring room, to monitor the patient 2, or may be processed by a device 8, e.g. a computer, to obtain a monitoring signal providing a particular information regarding the patient 2, e.g. if the patient 2 is still in the bed 3, the respiration rate or pulse rate of the patient 2, any activity of the patient 2, etc.

As shown in the scenario depicted in Fig. 1, the view of the imaging unit 5 can be hampered by several factors, such as an improper position of the patient 2 and/or the bed 3 in relation to the field of view 6 of the imaging unit 5, occlusions caused by objects 7 (such as a monitor or movable table) in between imaging unit 5 and patient 2 and occlusions caused by other people 4 (e.g. medical staff) in between imaging unit 5 and patient 2.

Known methods to address these challenges include visually checking the imaging unit's output and manually taking corrective action (e.g. manually changing the position or field of view of the imaging unit), which however has privacy concerns and requires skill and effort. Another known method is to provide specific workflow instructions on where to place the bed, objects, etc., which are however prone to human error and not very flexible. Further, redundancy may be provided through multiple imaging units involving however additional costs and increased system complexity.

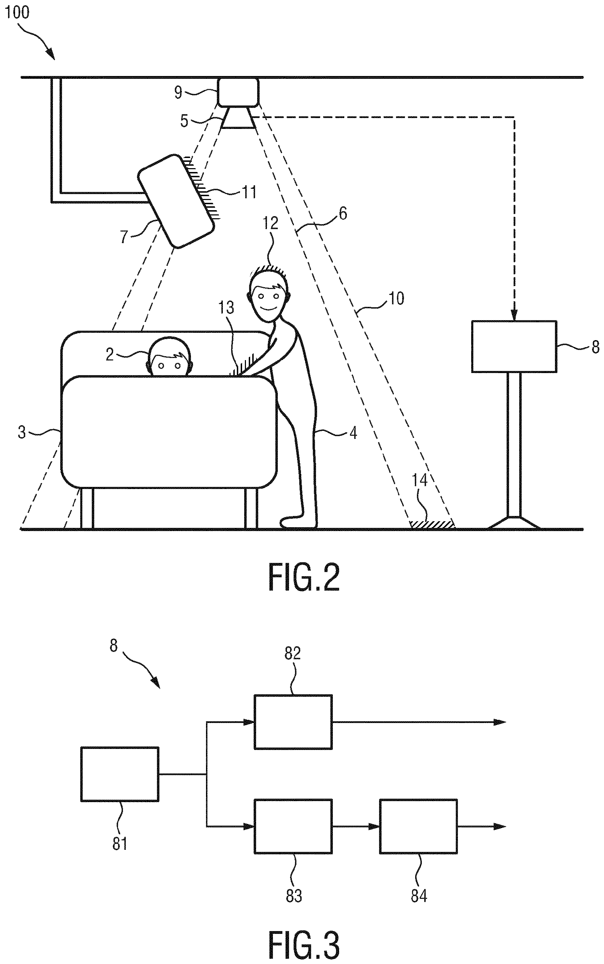

Fig. 2 shows a schematic diagram of a first embodiment of a system 100 according to the present invention. Generally, the scenario is similar as in Fig. 1, but the field of view 6 is at least temporarily illuminated, in this embodiment by use of a projection unit 9 (e.g. projector or a light source emitting a directed light beam) that is configured to at least temporarily illuminate the field of view 6 of the imaging unit 5. For this purpose, the (optional) projection unit 9 provided in this embodiment has a field of projection 10 that substantially overlaps with the field of view 6 of the imaging unit 5 and where the parallax is sufficiently small. Preferably, the projection unit 9 is mounted at, within or next to the imaging unit 5 to ensure that the field of view 6 and the field of projection 10 are arranged with sufficient overlap, in particular that the field of projection 10 completely covers the field of view 6.

Through the temporal illumination of the field of view 6, feedback about the spatial configuration of the patient 2 with respect to the imaging unit 5 can be provided. For instance, light patterns and/or shadows can be created using the projection unit 9, which informs e.g. medical staff about any problems with the spatial configuration. Such light patterns are exemplarily shown in Fig. 2, where light patterns 11, 12 and 13 indicate that objects 7 or other persons 4 may lead to occlusions of at least part of the patient 2 and light pattern 14 indicates that a part of the field of projection 10 is out of the field of view 6. In another embodiment a projected shape may be provided that corresponds to the field of view 6, implicitly indicating boundaries (seen as edges) and occlusions (seen as shadows). In still another embodiment specific patterns may be projected to indicate issues as detected by automatic analysis of the image data, e.g. part of the bed out of view or occlusion areas.

As shown in Fig. 2, the projection can be made directly onto the object/person causing a problem and/or the adjacent bed region to indicate both the cause (i.e. the object/person) and the effect (shadow). In another embodiment the projection can be used to indicate through illumination of the field of view if the spatial configuration can be maintained or shall be changed, e.g. by use of a color code where one color (e.g. red) indicates that changes are needed and another color (e.g. green) indicates that no changes are needed. Still further, in an embodiment specific instructions may be projected for corrective actions in the form of signs, symbols etc. in appropriate areas, e.g. an arrow on the bed in the direction where it should be moved, possibly with a distance indication. The arrow may start at the edge of the bed's current position and ends at the position where the bed should be. Further, by rendering arrows in the direction the user should move an object to reduce occlusion. The arrow starts at the edge of the object's current position and ends at the position where the object no longer occludes the region of interest.

According to the present invention, the imaging unit 5 may be a 2D optical camera (e.g. an RGB video camera, a webcam, or a still image camera that regularly acquires images), but may alternatively be an infrared camera (e.g. to work in darkness as well) or a 3D (depth) camera, such as a time-of-flight camera to provide distance information as e.g. used for monitoring the patient's activity as required in applications related to delirium detection or an ultrasound imaging device. The desired state is that the patient 2 on the bed 3 is fully within the field of view of the imaging unit 5.

In case a 3D (depth) camera is used as imaging unit (or if a 2D camera and a depth measurement unit, e.g. a radar unit, are used) depth information is available within or along with the image data. From this depth information (and the image data) it is possible to detect information about the spatial relationship in 3D, in particular to detect occlusions and occluding objects. A projection unit and temporal illumination of the field of view (during the image acquisition) in order to obtain information for detecting occlusions and occluding objects may in such an embodiment be omitted. Such an embodiment may hence be configured as shown in Fig. 1, with the difference that the imaging unit 5 is a 3D (depth) camera or that an image measurement unit (not shown) is provided in addition to a 2D camera.

The device 8 according to the present invention processes the image data acquired by the imaging unit 5 and generates a feedback signal providing the above described feedback. Fig. 3 shows a schematic diagram of another embodiment of a device 8 according to the present invention. It comprises an input interface 81 that obtains (i.e. receives or retrieves) image data acquired over time by the imaging unit 5 from the field of view 6 containing at least part of the subject 2 and being at least temporarily illuminated.

A monitoring signal determination unit 82 determines a monitoring signal from the obtained image data of the subject. The monitoring signal may e.g. be a PPG signal, a vital sign signal (e.g. a respiration signal, a pulse rate signal, an SpO2 signal, etc.), a motion signal indication motion or activity of the patient, etc.

In parallel, a processing unit 83 evaluates the image data to determine the spatial configuration of the subject 2 with respect to the imaging unit 5 and generates a feedback signal for providing feedback about the determined spatial configuration. Spatial configuration shall hereby be understood as the relative location and/or orientation of the subject 2 and the imaging unit 5 including information if and/or which parts of the subject that are at least potentially within the field of view 6 of the imaging unit 5 can actually not be "seen" (i.e. detected) by the imaging unit and are hence not depicted in the image data acquired by the imaging data.

An output interface 84 outputs the feedback signal (and optionally the monitoring signal).

The input interface 81 and the output interface 84 may be separate interfaces or a common interface. They may be implemented as conventional data interfaces for exchanging data e.g. via a wireless or wired network or directly with another device using a wired or wireless data communication technology (e.g. Wi-Fi, Bluetooth, LAN, etc.). The monitoring signal determination unit 82 and the processing unit 83 may be separate processing elements or a common processing element, such as a processor or a computer.

By use of the feedback signal a user (e.g. medical staff) gets information if and/or where problems might exist with respect to the current location of the imaging unit 5, the subject 2 and other objects or persons that might be present in the field of view 6 so that the user can take appropriate action to resolve the problem. In other embodiments the problem may even be resolved automatically as will be explained below.

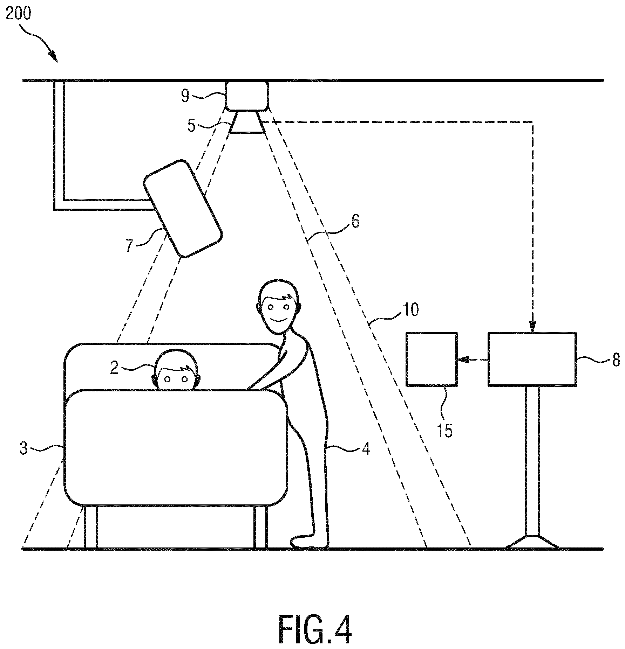

Fig. 4 shows a schematic diagram of a second embodiment of a system 200 according to the present invention. In this embodiment the system 200 comprises a user interface 15 that is configured to indicate through user feedback if the spatial configuration can be maintained or shall be changed. Further information may be provided as well by the user interface 15.

A possible feedback provided by the user interface 15 could be based on a display providing a color coding (i.e. like a traffic light), based on the amount of area that is in the field of view 6 of the imaging unit 5. For instance, red may indicate no bed and/or patient is in the field of view 6 (or that the region of interest determined from the image data is out of the field of view) and that the position of the bed and/or patient shall be changed. Yellow may indicate a partial occlusion (or that the region of interest is partly outside the field of view) and green may indicate that the bed and the patient are fully detectable and that there are no occlusions (from people and/or objects) (or that the region of interest is fully within the field of view).

Further, in an embodiment the field of view 6 may be indicated by rendering its outline (or filling its whole area) in a user-specified color (e.g. blue), the region of interest within the field of view 6 may be indicated by rendering its outline (or filling its whole area) in another user-specified color (e.g. green) and the region of interest outside the field of view 6 may be indicated by rendering its outline (or filling its whole area) in still another user-specified color (e.g. red). Further, objects/persons occluding the region of interest may be indicated by rendering their outlines (or filling their whole areas) in another user-specified color (e.g. orange).

Another possible feedback provided by the user interface 15 could be based on a loudspeaker providing an audio feedback, such as different sounds and/or alarms in relation to different types of occlusion (people, objects, partial occlusion, complete occlusion, etc.). For instance, a user-specified sound may be rendered if an object is occluding the region of interest. The quality of the spatial setup may be indicated by an acoustic traffic light with user-specified sounds for green, yellow and red as described above. Further, corrective actions may be supported by acoustic feedback (similar to acoustic parking assistance systems in cars), e.g. by use of a continuous tone if the bed is completely outside of the field of view, a slower tone sequence if the bed is going into the field of view, and no tone if the bed is completely inside the field of view. In another embodiment, a continuous tone may be rendered if the whole object is occluding the region of interest, a lower tone sequence may be rendered if less parts of the object are occluding the region-of-interest and no tone may be rendered if the object is no longer occluding the region of interest.

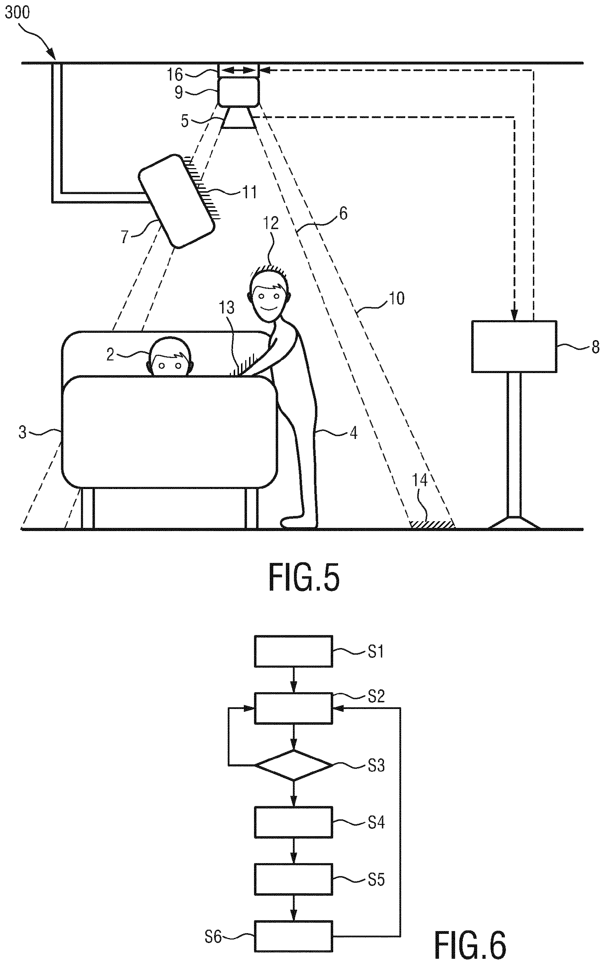

Fig. 5 shows a schematic diagram of a third embodiment of a system 300 according to the present invention. In this embodiment the system 300 comprises a means for changing the position and/or orientation of the imaging unit 5 and/or the bed 3. For instance, a robotic arm 16 or a motorized mount unit may be provided to which the imaging unit 5 (and the projection unit 9) are mounted. In response to a control signal generated and provided by the device 8 the robotic arm 16 may (automatically) change the position and/or orientation (i.e. tilt and/or pan) of the imaging unit 5 (with linear and/or rotational movements) if it is determined by the device 8 that the current spatial configuration of the imaging unit 5 causes problems related to the monitoring of the subject 2. Similarly, a motor (not shown) may be provided at the bed 3 to (automatically) change its position and/or orientation in response to a corresponding control signal from the device 8. With such an embodiment, the working space, the accuracy and the speed can be increased.

Fig. 6 shows a flow chart of an exemplary implementation of a method according to the present invention. In a first step S1 a user (e.g. nurse) rolls the bed with patient into the patient room in the general area of the imaging unit. In a second step S2 the processing unit 83 is triggered by the changed images from the imaging unit 5 to start analyzing the spatial configuration and compare to a desired state. In step S3 it is checked if the desired state is achieved. As long as the desired state is not achieved, the processing unit 83 determines appropriate feedback actions in step S4; otherwise the process loops back to step S2. In step S5 indications for appropriate feedback actions are sent to the (mount / visual / acoustic) feedback unit. In step S6 feedback actions are performed according to received indications, either automatically (e.g. by a motorized mount unit) and/or manually by a user (e.g. a nurse). The loop then goes back to step S2.

In an embodiment the user can configure the system, i.e. specify which of all the possible options mentioned above should be enabled or disabled.

The present invention is primarily used in contactless patient monitoring applications making use of cameras. In principle, however, it can be applied in many other camera-based applications. Further, it may be applied in X-ray systems to ensure that the right body parts are in the field of exposure (representing the field of view of the imaging unit) of the X-ray beam (representing the at least temporal illumination) emitted by the X-ray source and detected by an X-ray detector (representing the imaging unit), where one or more body parts to be X-rayed represent the region of interest.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for monitoring a subject, the device comprising:
- an input interface (81) configured to obtain image data acquired over time by an imaging unit from a field of view containing at least part of a subject,
- a monitoring signal determination unit (82) configured to determine a monitoring signal from the obtained image data of the subject,
- a processing unit (83) configured to evaluate the image data to determine the spatial configuration of the subject with respect to the imaging unit and to generate a feedback signal for providing feedback about the determined spatial configuration, and
- an output interface (84) configured to output the feedback signal.

2. Device as defined in claim 1,
wherein the processing (83) is configured to evaluate the image data to detect if the subject is visible in the image data or if the subject is occluded or out of the field of view of the imaging unit.

3. Device as defined in any one of the preceding claims,
wherein the field of view is at least temporarily illuminated from substantially the same direction, from which the imaging unit views the field of view.

4. Device as defined claim 3,
wherein the field of view is at least temporarily illuminated by a projection unit (9) having a field of projection that substantially overlaps with the field of view of the imaging unit.

5. Device as defined in any one of the preceding claims,
wherein the processing unit (83) is configured to generate the feedback signal that is configured to control a projection unit (9) to indicate through illumination of the field of view if the spatial configuration can be maintained or shall be changed.

6. Device as defined in any one of the preceding claims,
wherein the processing unit (83) is configured to generate the feedback signal that is configured to control a user interface (15) to indicate through user feedback if the spatial configuration can be maintained or shall be changed.

7. Device as defined in any one of claims 5 or 6,
wherein the processing unit (83) is configured to generate the feedback signal that is configured to control the projection unit and/or the user interface to indicate how the spatial configuration shall be changed.

8. Device as defined in any one of claims 5 or 6,
wherein the processing unit (83) is configured to generate the feedback signal that is configured to control the projection unit and/or the user interface to indicate:
if and/or how an occluding object shall be moved and/or,
if and/or how the subject and/or a support of the subject shall be moved and/or,
if and/or how the imaging unit and/or the projection unit shall be moved.

9. Device as defined in claim 5,
wherein the processing unit (83) is configured to generate the feedback signal that is configured to control the projection unit to indicate if the spatial configuration can be maintained or shall be changed by use of illumination of selected areas in the field of view and/or projection of one or more of symbols, colors, letters, and text.

10. Device as defined in claim 6,
wherein the processing unit (83) is configured to generate the feedback signal that is configured to control the user interface if the spatial configuration can be maintained or shall be changed by use of visual and/or audio feedback.

11. Device as defined in any one of the preceding claims,
wherein the processing unit (83) is configured to generate a control signal for controlling the imaging unit and/or a support for supporting the subject based on the determined spatial configuration to change its position and/or orientation in order to change the spatial configuration, and
wherein the output unit (84) is configured to output the control signal.

12. System for monitoring a subject, the system comprising:
- an imaging unit (5) configured to acquire image data over time from a field of view (6) containing at least part of a subject, and
- a device (8) defined in any one of the preceding claims for determining a monitoring signal of a subject based on the acquired image data.

13. System as claimed in claim 12, further comprising
- a projection unit (9) configured to at least temporarily illuminate the field of view, the projection unit (9) having a field of projection (10) that substantially overlaps with the field of view of the imaging unit, and/or to indicate through illumination of the field of view if the spatial configuration can be maintained or shall be changed, and/or
- a user interface (15) configured to indicate through user feedback if the spatial configuration can be maintained or shall be changed.

14. Method for monitoring a subject, the method comprising:
- obtaining image data acquired over time by an imaging unit from a field of view containing at least part of a subject,
- determining a monitoring signal from the obtained image data of the subject,
- evaluating the image data to determine the spatial configuration of the subject with respect to the imaging unit,
- generating a feedback signal for providing feedback about the determined spatial configuration, and
- outputting the feedback signal.

15. Computer program comprising program code means for causing a computer to carry out the steps of the method as claimed in claim 14 when said computer program is carried out on the computer.
